# EUROPEAN PATENT APPLICATION

(11) **EP 3 915 556 A1**
(43) Date of publication of application: **01.12.2021**
(21) Application number: 20176865.2
(22) Date of filing: 27.05.2020
(51) Int. Cl.: A61K 31/4035, A61K 9/00, A61K 31/454, A61P 1/00, A61P 1/04, A61P 43/00

(54) **RECTAL APPLICATION OF THALIDOMIDE IN PATIENTS WITH HEMORRHAGIC CHRONIC RADIATION PROCTITIS**

(71) Applicant: Vonnahme, Franz Josef, 31787 Hameln (DE)
(72) Inventor: Vonnahme, Franz Josef, 31787 Hameln (DE)
(74) Representative: Lederer & Keller Patentanwälte Partnerschaft mbB

(57) **Abstract**

Hemorrhagic chronic radiation proctitis is a long-term side effect of radiotherapy in the treatment of pelvic malignancies. The present invention pertains to the therapeutic use of thalidomide or an analogue thereof for treating hemorrhagic chronic radiation proctitis, wherein said treatment comprises rectally administering said thalidomide or its analogue to a patient in need thereof. Topical treatment of the areas affected by hemorrhagic chronic radiation proctitis with thalidomide leads to a long lasting decay of the hemorrhagic chronic radiation proctitis and healing of the affected tissue.

## Description

### FIELD OF THE INVENTION

As a late consequence of pelvic radiotherapy profuse bleedings from the rectum may occur. Invasive therapy methods are used to achieve hemostasis, which after thermal or chemical intervention (argon plasma coagulation, formalin instillation) cause damage of the bleeding mucosa followed by scar formation. In most cases these interventions do not lead to a final recovery but side effects prevail. Treatment approaches with various medications have so far not been convincing. The present invention provides a new treatment option for late bleeding events resulting from pelvic radiotherapy.

### BACKGROUND

One of the prevalent therapy options for various kinds of malignancies is ionising radiation (radiotherapy). Patients with urological, gynaecological and gastrointestinal malignancies, in areas such as the prostate, urinary bladder, cervix, uterus and anus can be treated with external transcutaneous radiation or internal brachytherapy. The radiation dose is applied locally to minimize side effects, but tissue damage in the neighbouring organs is unavoidable. The irradiated tissue regenerates very poorly or not at all (Jongen J, et al. Proktitis aus Sicht der Proktologie. Coloproctology 32: 273-278). Radiation dose and radiation type directly influence the degree of the tissue damage with short and long term side effects observable (Dahm K, et al. STRAHLENINDUZIERTE STENOSEN DES SIGMA UND REKTUMS, Chir Prax 21, 1979).

Acute radiation proctitis occurs within a very short latency period after or even during radiotherapy (short term side effect). Symptoms are rectal bleeding, mucus secretion, diarrhoea, urge to defecate, incontinence, a thickened mucosa around the affected areas, erosive or ulcerative lesions, polyposis or even tumorous changes, telangiectasia and atypical fistula openings. They are a direct consequence of the physical damage due to the radiation (Jongen J, et al. Proktitis aus Sicht der Proktologie. Coloproctology 32: 273-278).

Pelvic radiotherapy associated late effects are often referred to as chronic radiogenic proctitis or chronic radiation proctitis. The term "proctitis" is somewhat misleading, as it suggests that this condition involves inflammation which, however, is usually not the case. In contrast to acute radiation proctitis, chronic radiation proctitis typically is not an inflammatory condition. Therefore, some authors prefer the term chronic radiation proctopathy (WU X, et al. Pathogenesis, Diagnosis, and Management of Ulcerative Proctitis, Chronic radiation Proctopathy, and Diversion Proctitis. Inflamm Bowl Dis 2015; 21:703-715). In some publications the symptoms are classified as "inflammation-pre-dominant form", "bleeding-predominant form" and "mixed form". The late effects of pelvic radiation as characterised below are referred to hereinafter as hemorrhagic chronic radiation proctitis.

Hemorrhagic chronic radiation proctitis typically develops approximately 3-9 months after radiotherapy. (Lucarotti ME, et al. Surgical management of intestinal radiation injury, Dis Colon Rectum, 1991;34:865-869, Vanneste B, et al. Chronic radiation proctitis: tricks to prevent and treat, Int J Colorectal Dis (2015) 30:1293-1303). At radiation doses between 45-55 Gy, 5% of the patients develop symptoms within 5 years. From 60 Gray (Gy) upward the occurrence of side effects such as hemorrhagic chronic radiation proctitis drastically increases (Roswit B, et al. SEVERE RADIATION INJURIES OF THE STOMACH SMALL; INTESTINE; COLON AND RECTUM. Amer J Roentenol 114 (1972), 460). Up to 20 % of irradiated patients are affected (Vanneste B, et al. Chronic radiation proctitis: tricks to prevent and treat, Int J Colorectal Dis (2015) 30:1293-1303).

The main symptom of hemorrhagic chronic radiation proctitis is rectal bleeding which can lead to iron-deficiency and anaemia with the patient requiring blood transfusion. Major treatment aim in hemorrhagic chronic radiation proctitis therapy is to stop the bleeding. The origin of the bleeding is a circumscribed area of the rectal mucosa adjacent to the radiated organ. The radiation results in alterations of the microvessels of the rectal mucosa predominantly in the anterior rectal wall. In rectal biopsies from patients suffering from radiation proctitis it could be shown that angiogenin and FGF1 (fibroblast growth factor) promoted endothelial cell proliferation and endoglin induced vessel formation cumulating in angiogenesis (Takeuchi H. et al, A Mechanism for Abnormal Angiogenesis in Human Radiation Proctitis: Analysis of Expression Profile for Angiogenic Factors, J Gastroenterol 2012, 47(1), 56-64). Cellular basis of hemorrhagic chronic radiation proctitis is a mutation-related proliferation of endothelial cells as a consequence of the radiotherapy. Radiation-related mutations of the vascular cells lead to the development of fragile teleangiectatic vessels. The increased growth of vascular cells leads to capillary ectasias and the forming of vascular convolutes (angiodysplasias). The vascular walls of these pathological capillaries do not mature, they do not seal up and capillary bleeding occurs. Vascular endothelial growth factor (VEGF) as an indicator for angiogenesis is significantly increased in patients with hemorrhagic chronic radiation proctitis (Trzcinski R, et al. Expression of vascular endothelial growth factor and its correlation with clinical symptoms and endoscopic findings in patients with chronic radiation proctitis, Colorectal Dis. 2018 Apr; 20(4):321-330). Microvascular density increased significantly 6 months after radiotherapy. This observation correlated with an increase of VEGF at the completion of radiotherapy (Karamanolis G et al, Increased Expression of VEGF and CD31 in Postradiation Rectal Tissue: Implications for Radiation Proctitis, Hindawi Publishing Corporation, Mediators of Inflammation, Vol 2013, Article ID 515048, 7 pages, 2013; http://dx.doi.org/10.1155/2013/515048).

Many treatment attempts with varying degree of success have been reported for hemorrhagic chronic radiation proctitis. As there is no standard therapy, published reports present very small patient groups or single cases. The treatment options can be divided into pharmacotherapy and endoscopic treatments (Nelamangala Ramakrishnaiah VP, Krishnamachari S, Chronic Haemorrhagic Radiation Proctitis: A Review, World J Gastrointest Surg, 2016, 27, 8(7), 483-491).

Pharmacotherapies (applied topically as a cream or enema) are for example 5-aminosalicylic acid (5-ASA, active form of sulfasalazine), sucralfate, corticosteroid, formalin and short chain fatty acids (SCFAs) (WU X, et al. Pathogenesis, Diagnosis, and Management of Ulcerative Proctitis, Chronic radiation Proctopathy, and Diversion Proctitis. Inflamm Bowl Dis 2015; 21:703-715). Topical or oral 5-ASA has been suggested to be a promising agent in some reports, but results were not reproducible (BAUM CA, et al. Failure of 5-aminosalicylic acid enemas to improve chronic radiation proctitis. Dig Dis Sci. 1989; 34:758-760). Topical formaldehyde is accompanied by significant bleeding and necrosis of the coagulated tissue on contact. Additionally, serious complications such as fistula development requiring colostomy as well as bowel necrosis requiring resection have been reported (de Parades V, et al. Formalin application in the treatment of chronic radiation-induced hemorrhagic proctitis - an effective but not risk-free procedure: a prospective study of 33 patients. Dis Colon Rectum. 2005;48:1535-1541; LUNA-PEREZ P, et al. Formalin instillation for refractory radiation-induced hemorrhagic proctitis. J Surg Oncol 2002; 80:41-44). Contrary to initial supposition, SCFA enemas have not been found to be effective (TALLEY NA, et al. Short-Chain fatty acids in the treatment of radiation proctitis: a randomized, double blind, placebo controlled, crossover pilot trial. Dis Colon Rectum. 1997; 40:1046-1050.). Hyperbaric oxygen therapy may further improve the symptoms of hemorrhagic chronic radiation proctitis but it is expensive, time consuming and suitable pressure chamber facilities very limited in availability (CLARKE RE, et al. Hyperbaric oxygen treatment of chronic refractory radiation proctitis: a randomized and controlled double blind crossover trial with long-term follow-up. Int J Radiat Oncol Biol Phys. 2008;72:134-1439). Currently, argon plasma coagulation (APC) is the most promising treatment for hemorrhagic chronic radiation proctitis. It can decrease the bleeding but is not applicable when larger tissue areas are affected. Prevalent side effects are ulcers, strictures and rectovaginal fistulas. (RUSTAGI T, MASHIMO H. Endoscopic management of chronic radiation proctitis. World J Gastroenterol. 2011; 17:4554-4562). Neodym:YAG (neodymium-doped yttrium aluminium garnet) laser can also coagulate bleeding vessels. It is however expensive and associated with a high risk of injury during use. Fibrosis, stricture formation and transmural inflammation are commonly reported as side effects (LEIPER K, MORRIS Al, Treatment of radiation proctitis, Clin Oncol (R Coll Radiol), 2007;19:724-729).

The various attempts to standardize hemorrhagic chronic radiation proctitis treatment and to find new treatment options clearly show that the currently available therapy options are not satisfactory as they are mostly managing but not curing the pathology, accompanied by a high risk of severe side effects (TABAJA L, SIDANI SM. Management of Radiation Proctitis. Dig Dis Sci. 2018 Sep; 63(9):2180-2188). A treatment that reliably leads to an elimination of the symptoms and allows the patient to lead a life independent of medication is not available so far.

The formation of new vessels can be inhibited by the anti-angiogenic effect of thalidomide. Brought to the market as a sedative, thalidomide is nowadays used in the treatment of various diseases. Due to its tumour growth inhibiting effects, it is used in patients suffering from multiple myeloma. Typical side effects of thalidomide are sedation and neuropathy which often leads to a discontinuation of the therapy (Mileshkin L, et al. Development of neuropathy in patients with myeloma treated with thalidomide: pattern of occurrence and the role of electrophysiologic monitoring, J Clin Oncol. 2006 Sep 20; 4507-14). In individual cases of patients with hemorrhagic radiation proctitis, oral thalidomide as tablets could stop the bleeding. However, due to known side effects of thalidomide (sedation, rash, dizziness, headache, mood changes, tremor liver toxicity (or even acute liver failure), fatigue, constipation, polyneuropathy, thrombosis) after oral administration, higher doses and long-term use in other indications, the therapy with the substance in chronic radiation proctitis was not pursued further. Furthermore, bleedings often reoccurred after thalidomide termination (Izquierdo Navarro et al., Farm Hosp, 2016, Vol. 40(3):230-232.; McFarlane et al., Frontline Gastroenterology, 2018; Vol. 9(2): 98-104.).

### SUMMARY OF THE INVENTION

The inventor surprisingly found that hemorrhagic chronic radiation proctitis can be successfully treated by rectal application of relatively low doses of thalidomide. Surprisingly, bleedings stopped already few days after the start of the treatment. No adverse side effects were observed. No bleedings reoccurred. In particular, the adverse effects such as neuropathy reported in connection with oral administration of thalidomide did not occur. The rectal application of thalidomide allows using relatively low doses of the drug, whereby systemic side effects can be avoided.

The present invention therefore relates to the subject matter defined in the following items 1 to 56:
[1] A compound selected from the group consisting of thalidomide, thalidomide analogues and salts thereof, for use in the treatment of hemorrhagic chronic radiation proctitis, wherein said treatment comprises rectally administering said compound to a patient.
[2] The compound for use according to item 1, wherein said hemorrhagic chronic radiation proctitis is a non-inflammatory condition.
[3] The compound for use according to any one of the preceding items, wherein the treated patient had suffered and or is suffering from prostate cancer, cervical cancer or uterine cancer.
[4] The compound for use according to any one of the preceding items, wherein the treated patient has an age of at least 18 years, or at least 35 years, or at least 50 years, or at least 65 years.
[5] The compound for use according to any one of the preceding items, wherein the first occurrence of the hemorrhagic chronic radiation proctitis was at least 8 weeks after radiotherapy.
[6] The compound for use according to any one of the preceding items, wherein the first occurrence of the hemorrhagic chronic radiation proctitis was at least 12 weeks after radiotherapy.
[7] The compound for use according to any one of the preceding items, wherein the first occurrence of the hemorrhagic chronic radiation proctitis was at least 6 months after radiotherapy.
[8] The compound for use according to any one of the preceding items, wherein the hemorrhagic chronic radiation proctitis was diagnosed at a time which is from 8 weeks to 60 months after radiotherapy.
[9] The compound for use according to any one of the preceding items, wherein the hemorrhagic chronic radiation proctitis was diagnosed at a time which is 3 to 36 months after radiotherapy.
[10] The compound for use according to any one of the preceding items, wherein the hemorrhagic chronic radiation proctitis was diagnosed at a time which is 6 to 24 months after radiotherapy.
[11] The compound for use according to any one of the preceding items, wherein the hemorrhagic chronic radiation proctitis occurred within 18 months after radiotherapy.
[12] The compound for use according to any one of the preceding items, wherein the hemorrhagic chronic radiation proctitis occurred within 24 months after radiotherapy.
[13] The compound for use according to any one of the preceding items, wherein the hemorrhagic chronic radiation proctitis occurred within 36 months after radiotherapy.
[14] The compound for use according to any one of the preceding items, wherein the hemorrhagic chronic radiation proctitis occurred within 360 months after radiotherapy.
[15] The compound for use according to any one of the preceding items, wherein the compound is a pharmaceutically acceptable prodrug, salt, solvate, or clathrate of thalidomide or of its analogue.
[16] The compound for use according to any one of items 1 to 14, wherein said compound is thalidomide.
[17] The compound for use according to any one of the preceding items, wherein the patient is receiving anticoagulants.
[18] The compound for use according to any one of the preceding items, for use as a first-line therapy in the treatment of hemorrhagic chronic radiation proctitis.
[19] The compound for use according to any one of items 1 to 17, wherein said treatment further comprises applying a different therapy for treating hemorrhagic chronic radiation proctitis.
[20] The compound for use according to item 19, wherein said different therapy comprises argon plasma coagulation.
[21] The compound for use according to item 20, wherein said argon plasma coagulation therapy was terminated prior to administering the compound.
[22] The compound for use according to any one of the preceding items, wherein the patient also receives or has received hormone therapy.
[23] The compound for use according to any one of the preceding items, wherein said administering comprises or consists of administering a composition comprising the compound and at least one pharmaceutically acceptable excipient.
[24] The compound for use according to item 23, wherein the composition is a solution, suspension, spray, pastille, foam, emulsion, ointment, cream, paste, suppository, enema, or gel.
[25] The compound for use according to item 23 or 24, wherein the composition is a solution.
[26] The compound for use according to item 23 or 24, wherein the composition is a suspension.
[27] The compound for use according to item 23 or 24, wherein the composition is a spray.
[28] The compound for use according to item 23 or 24, wherein the composition is a foam.
[29] The compound for use according to item 23 or 24, wherein the composition is an emulsion.
[30] The compound for use according to item 23 or 24, wherein the composition is an enema.
[31] The compound for use according to item 23 or 24, wherein the composition is an ointment.
[32] The compound for use according to item 23 or 24, wherein the composition is a cream.
[33] The compound for use according to item 23 or 24, wherein the composition is a paste.
[34] The compound for use according to item 23 or 24, wherein the composition is a suppository.
[35] The compound for use according to item 23 or 24, wherein the composition is a gel.
[36] The compound for use according to any one of the preceding items, wherein the compound is administered at a dose from 5 mg per day up to 100 mg per day.
[37] The compound for use according to any one of the preceding items, wherein the compound is administered at a dose from 15 mg per day up to 80 mg per day.
[38] The compound for use according to any one of the preceding items, wherein the compound is administered at a dose from 20 mg per day up to 75 mg per day.
[39] The compound for use according to any one of the preceding items, wherein the compound is administered at a dose from 25 mg per day up to 70 mg per day.
[40] The compound for use according to any one of the preceding items, wherein the compound is administered at a dose from 30 mg per day up to 65 mg per day.
[41] The compound for use according to any one of the preceding items, wherein the compound is administered at a dose from 10 mg per day up to 55 mg per day.
[42] The compound for use according to any one of items 1 to 35, wherein the compound is administered at a dose from 10 mg per day up to less than 50 mg per day.
[43] The compound for use according to any one of items 1 to 35, wherein the compound is administered at a dose from 10 mg per day up to 45 mg per day.
[44] The compound for use according to any one of items 1 to 35, wherein the compound is administered at a dose from 10 mg per day up to 40 mg per day.
[45] The compound for use according to any one of items 1 to 35, wherein the compound is administered at a dose from 10 mg per day up to 35 mg per day.
[46] The compound for use according to any one of items 1 to 35, wherein the compound is administered at a dose from 10 mg per day up to 30 mg per day.
[47] The compound for use according to any one of items 1 to 35, wherein the compound is administered at a dose from 10 mg per day up to 25 mg per day.
[48] The compound for use according to any one of the preceding items, wherein the treatment period lasts up to 7 days.
[49] The compound for use according to any one of the preceding items, wherein the treatment period lasts up to 10 days.
[50] The compound for use according to any one of the preceding items, wherein the treatment period lasts up to 14 days.
[51] The compound for use according to any one of the preceding items, wherein the treatment period lasts less than three weeks.
[52] The compound for use according to any one of the preceding items, wherein the treatment period lasts up to 21 days or up to 63 days.
[53] The compound for use according to any one of the preceding items, wherein the treatment period lasts 3 to 70 days, or from 4 to 56 days, or from 5 to 42 days, or from 6 to 28 days, or from 7 to 21 days, or from 4 to 14 days, or from 4 to 10 days.
[54] The compound for use according to any one of the preceding items, wherein the patient additionally receives anticoagulants.
[55] The compound for use according to any one of the preceding items, wherein the compound is administered once daily.
[56] A method of treating hemorrhagic chronic radiation proctitis, comprising rectally administering to a patient an effective amount of a compound selected from the group consisting of thalidomide, thalidomide analogues and pharmaceutically acceptable salts thereof.

### DESCRIPTION OF THE DRAWINGS

**Figure 1**: Pre-treatment endoscopic photography of affected rectal areas in patient suffering from hemorrhagic chronic radiation proctitis.
**Figure 2**: Post-treatment endoscopic photography of affected rectal areas six months after completed treatment with Thalidomide (same patient as figure 1).

### DETAILED DESCRIPTION

The present invention relates to a compound selected from the group consisting of thalidomide, analogues of thalidomide and salts thereof, for use in the treatment of hemorrhagic chronic radiation proctitis, wherein said treatment comprises rectally administering said compound to a patient.

### Hemorrhagic chronic radiation proctitis and treatment

The term "hemorrhagic chronic radiation proctitis", as used herein, refers to rectal bleeding occurring at least eight weeks after exposure to ionizing irradiation, e.g. in pelvic radiotherapy.

The major symptom of hemorrhagic chronic radiation proctitis is rectal bleeding. In severe cases, the bleeding leads to anaemia and patients then require blood transfusions. The rectal bleeding is a consequence of radiation-induced malformations of capillaries in the rectal mucosa.. The endothelial cells of the capillaries do not mature and continuously undergo proliferation cycles. This leads to a leaky vessel wall leading to diffuse bleeding.

A clear distinction is made herein between hemorrhagic chronic radiation proctitis described above and acute radiation proctitis. The latter refers to the acute effects of the irradiation used in radiotherapy. Acute radiation proctitis occurs soon after radiation treatment. Symptoms of acute radiation proctitis can be rectal bleeding, mucus secretion, diarrhoea, urge to defecate, incontinence, a thickened mucosa around the affected areas, erosive or ulcerative lesions, polyposis or even tumorous changes, telangiectasia and atypical fistula openings. Acute radiation proctitis is an inflammatory condition which may heal by forming thickened rectal wall and/or scars.

Hemorrhagic chronic radiation proctitis, however, in contrast to acute radiation proctitis, does not have inflammatory characteristics. The person skilled in the art will be able to differentiate between hemorrhagic chronic radiation proctitis and acute radiation proctitis.

Thalidomide has been reported to evoke severe side effects such as fatigue, constipation, polyneuropathy, thrombosis. Surprisingly, rectal application of thalidomide sufficiently stops the bleeding while side effects observed with high oral accumulative doses are not apparent. Patients that also take anticoagulants especially profit from the rectal application of thalidomide in the treatment of hemorrhagic chronic radiation proctitis.

In another embodiment of the current invention the hemorrhagic chronic radiation proctitis occurs at least 8 weeks after radiotherapy. That is, the first onset of the hemorrhagic chronic radiation proctitis is at least 8 weeks after radiotherapy. In another embodiment the hemorrhagic chronic radiation proctitis occurs up to 30 years after radiotherapy. In the most preferred embodiment of the current invention, the hemorrhagic chronic radiation proctitis occurs 6-24 months after radiotherapy.

In one embodiment, treated patients suffer from hemorrhagic conditions of the rectal mucosa. In one embodiment, the origin of the bleeding is a circumscribed area of the rectal mucosa that is adjacent to the prostate. In another embodiment of the current invention the treated patient suffers from angiodysplasias in the rectum and/or the tissue areas adjacent to the rectum.

In one embodiment the current invention can be used as a first-line therapy for hemorrhagic chronic radiation proctitis. In another embodiment, the current invention is used in combination with another treatment option for hemorrhagic chronic radiation proctitis. In one embodiment, the current invention is used after the patient received argon plasma coagulation therapy.

In one embodiment the treated patient had suffered and or is suffering from prostate cancer. In another embodiment the treated patient had suffered and or is suffering from cervical cancer. In yet another embodiment the treated patient had suffered and or is suffering from uterine cancer.

### Rectal application

In accordance with this invention the compound is administered rectally.

The compound can be administered in the form of a solution, suspension, spray, pastille, foam, emulsion, ointment, cream, paste, suppository, enema, or gel. Preferably, the composition to be administered is a suppository.

The compound may be supplied in unit doses, and suitable formulations can prepared by methods known in the art. This process typically comprises bringing together the active ingredient with a pharmaceutical carrier to form a unit dose. A single unit dosage form comprises the active ingredient or a pharmaceutical acceptable prodrug, salt, solvate or clathrate thereof; and at least one excipient. The single unit dose is preferably a daily dose or a daily sub-dose. The pharmaceutical carrier may be liquid and or solid. After contacting the pharmaceutical carrier and the active ingredient both are mixed together until the active ingredient is uniformly distributed within the pharmaceutical carrier, after which the resulting mixture may be shaped. In some embodiments, the rectal application of the active ingredient is via a suspension, solution, emulsion, gel or enema. The active ingredient is then delivered within a liquid which may exert hydrophobic or hydrophilic or amphiphilic characteristics. In one embodiment the rectal application is achieved with a rectal suppository. In this case the active ingredient is mixed with an appropriate base such as cocoa butter or a salicylate. Additionally to the active ingredient and the pharmaceutical carrier the unit dose of the present invention may comprise further agents conventional in the art with regard to the application route, and the chemical proposition of the active ingredient and or the pharmaceutical carrier.

The dosage of the compound will depend on medical factors such as the severity of the hemorrhagic chronic radiation proctitis, the overall state and health of the patient, the body weight, physical activity as well as weather the patient receives further medication. In one embodiment of the current invention the preferred daily dose of the compound is from 5 to 100 mg. In another embodiment of the current invention the daily dose is from 15 to 85 mg. In another embodiment of the current invention the daily dose is from 25 to 75 mg. In another embodiment of the current invention the daily dose is from 35 to 65 mg. In another embodiment of the current invention the daily dose is from 45 to 75 mg. In the most preferred embodiment of the current invention the daily dose is from 15 to 45 mg. The treatment may comprise administering the same dose each day over the complete treatment period. Alternatively the dose may be varied during the treatment period, e.g. by an alternating pattern of low doses and high doses. For example, the treatment may comprise administering a low dose on day 1, a high dose on day 2, a low dose on day 3, a high dose on day 4 etc. Different patterns can be contemplated as well. A low dose may range from 1 to 20 mg per day, a high dose may range from greater than 20 mg to 100 mg per day.

In one preferred embodiment of the current invention the dosage can be adjusted according to how well he patient responds to the therapy over the course of the treatment time. The ordinary person skilled in the art is well aware of how a suitable dosage can be achieved.

In one embodiment of the current invention the compound is administered consecutively every day, as long as the treatment continues. The overall treatment period depends on the severity of the hemorrhagic chronic radiation proctitis, the overall state and health of the patient, his or her body weight, physical activity as well as whether the patient receives further medication and is left for the person skilled in the art to decide what is advised to the patient. In another embodiment of the current invention the compound is administered every other day with a regular or irregular schedule. In the most preferred embodiment of the current invention the compound is applied consecutively every day throughout the treatment period. In one embodiment the treatment period lasts up to 7 days. In another embodiment the treatment period lasts up to 14 days. In one embodiment, the treatment period lasts up to 21 days or less than 21 days. In another embodiment the treatment period lasts up to 28 days. In one embodiment the treatment period lasts up to 35 days. In another embodiment, the treatment period lasts up to 42 days. In one embodiment lasts up to 49 days. In one embodiment of the current invention the treatment period lasts from one up to 63 days. In another embodiment of the current invention the treatment period lasts from seven days up to 56 days. In another embodiment of the current invention the treatment period lasts from 14 days to 49 days.

The current invention is particularly beneficial for patients who receive or received anticoagulants. This is based upon the fact that these patients exhibit a drug-induced inhibited blood coagulation. Rectal bleeding associated with hemorrhagic chronic radiation proctitis leads to a higher blood loss and delayed healing. If the patient is on treatment with anticoagulants, the treatment period of the present invention is preferably from 10 days to 35 days, or from 14 days to 28 days. If the patient does not receive anticoagulants, the treatment period of the present invention is preferably from 4 days to 20 days, or from 7 days to 14 days.

### The compound

The compound thalidomide is also known as α-Phthalimidoglutarimid or (RS)-2-(2,6-dioxopiperidin-3-yl)-1H-isoindole-1,3(2H)-dione. Thalidomide has the following structure:

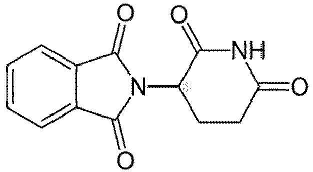

Most preferably the compound to be used is thalidomide. In other embodiments the compound is an analogue of thalidomide. Preferably, the analogue of thalidomide or a pharmaceutically acceptable salt thereof has anti-angiogenic activity. Anti-angiogenic activity can be determined in a known angiogenesis inhibition assay, e.g. in a HUVEC MTT Assay, a chicken embryo chorioallantoic membrane assay, rat aortic ring assay, or in a rabbit cornea angiogenesis assay. Preferably, anti-angiogenic activity is determined in a HUVEC MTT Assay as described in Example 1 of WO 2005/016326 A2. Preferred analogues of thalidomide include lenalidomide, pomalidomide, iberdomide and apremilast.

In one embodiment the analogue of thalidomide is a compound selected from the group consisting of 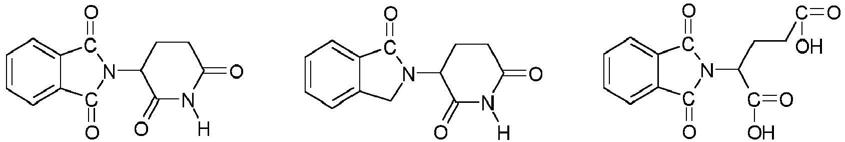 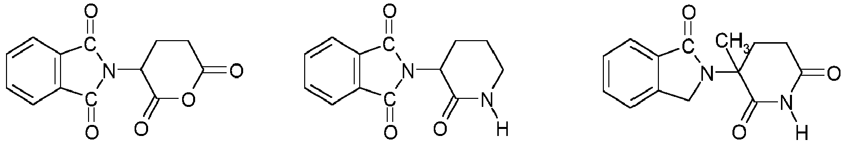 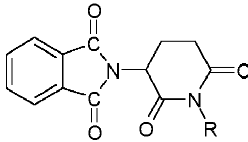 wherein R is -H, -OH, or -CH₃ 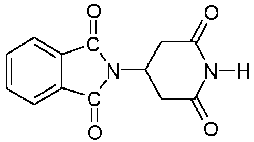 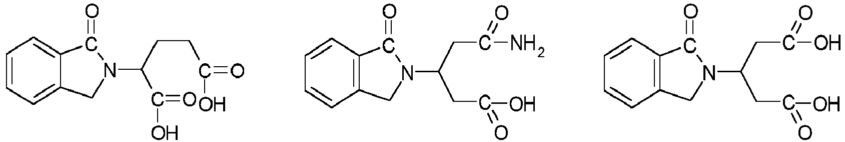 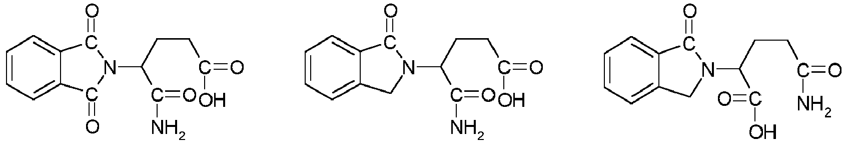 and

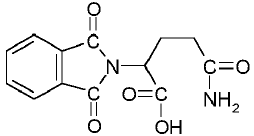

In another embodiment the analogue of thalidomide is a compound selected from the group consisting of

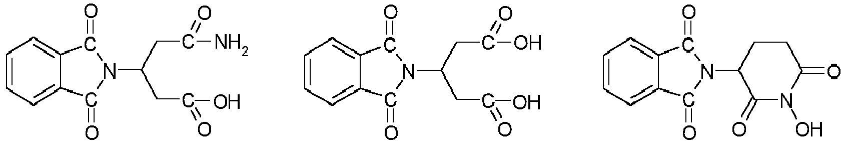

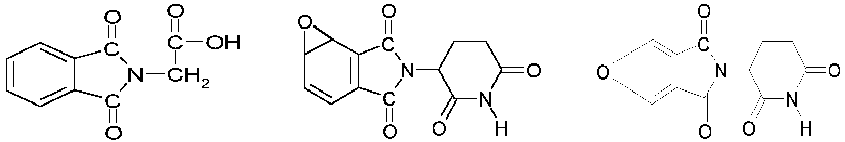

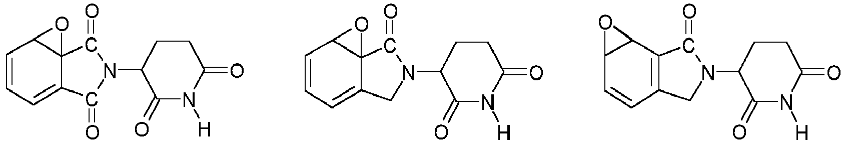

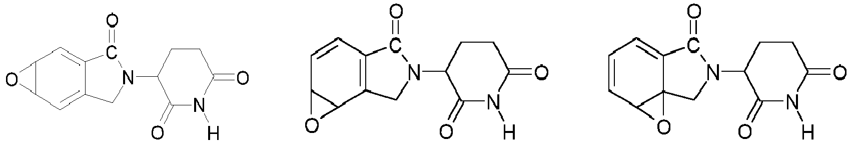

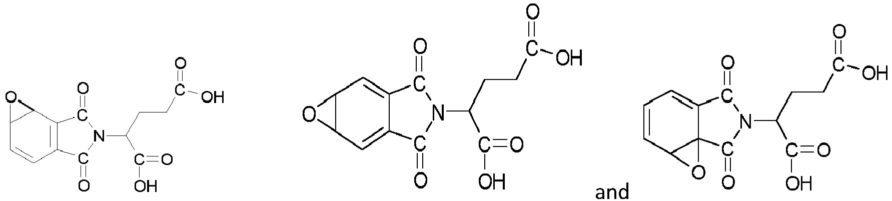

In another embodiment the analogue of thalidomide is a compound selected from the group consisting of

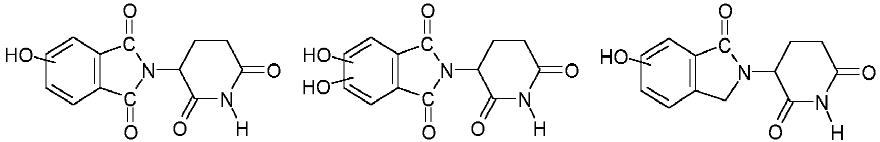

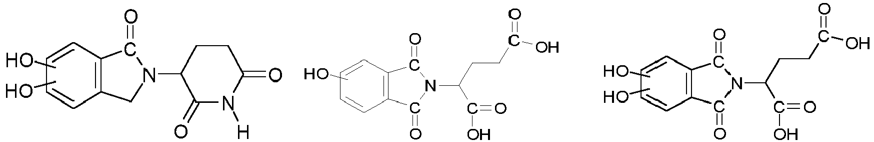

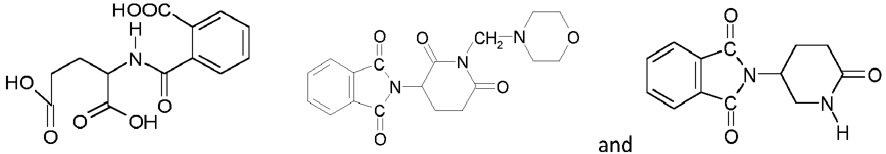

In another embodiment the analogue of thalidomide is a compound selected from the group consisting of 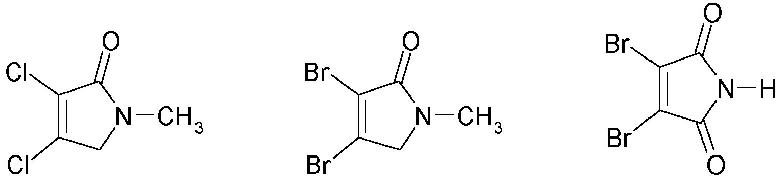 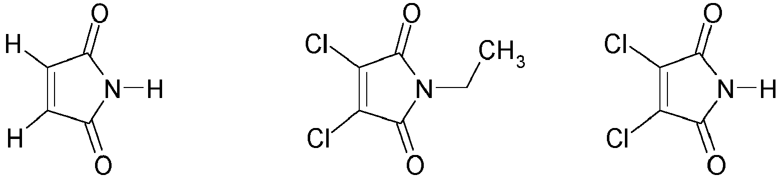 and

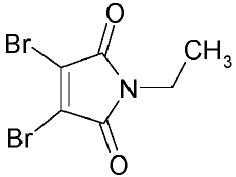

In another embodiment the analogue of thalidomide is a compound selected from the group consisting of

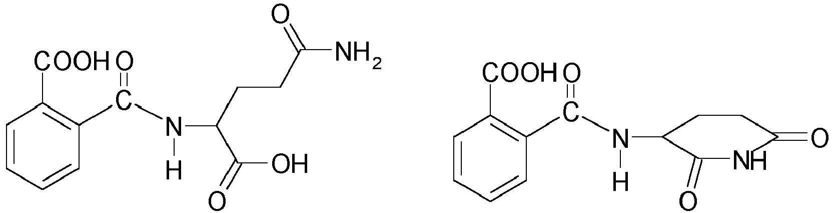

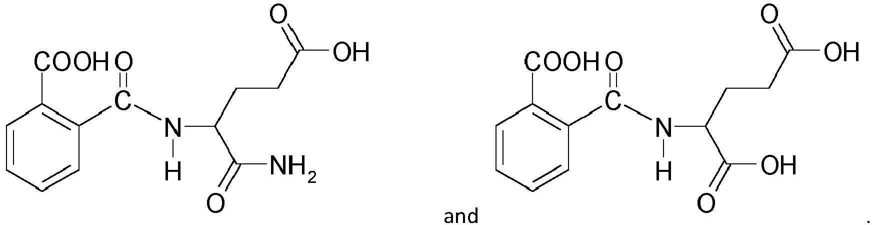

In summary, the preferred compounds are thalidomide, as well as analogues, hydrolysis products, metabolites and precursors of thalidomide that inhibit angiogenesis. It is to be understood that the compounds of the present invention can exist as enantiomers and that the racemic mixture of enantiomers or the isolated enantiomers are all considered as within the scope of the present invention. The compounds described above can be provided as pharmaceutically acceptable formulations using formulation methods known to those of ordinary skill in the art.

In the current invention - in place of or in combination with thalidomide- an analogue or pharmaceutically acceptable salt as defined above can be used. The thalidomide or combinations, analogues or pharmaceutically acceptable salt may be integrated into appropriate carrier(s) such as a biodegradable polymer for sustained release. Hereafter, when mentioning the active ingredient, the above mentioned combinations, analogues or pharmaceutically acceptable salts thereof are understood to be included likewise. A single unit dosage form comprises the active ingredient or a pharmaceutical acceptable prodrug, salt, solvate or clathrate thereof; and at least one excipient.

### EXAMPLE

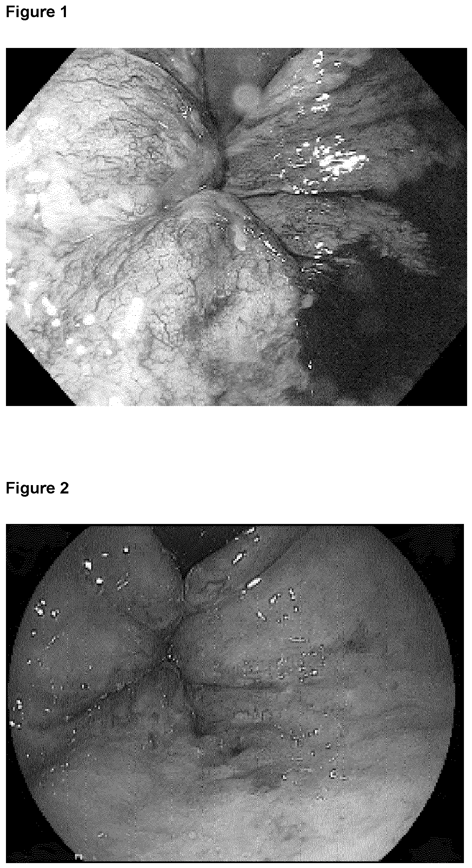

The rectal application of thalidomide has been investigated in patients suffering from hemorrhagic chronic radiation proctitis (Figure 1). Three patients aged 69, 81 and 82 developed persistent peranal bleeding one to two years after radiation. Endoscopically, the patients showed the typical picture of hemorrhagic chronic radiation proctitis. Two of the patients needed anticoagulation for atrial fibrillation. The patients received 20 mg thalidomide in a carrier material as a suppository once a day for a period of three weeks. The suppositories were manufactured by mixing together the appropriate amount of thalidomide with melted cocoa butter, pouring the mixture into a mould which is conventionally used for forming suppositories and allowing the mixture to harden at room temperature. The patient self-administered the suppositories once a day for up to 21 days.

Treatments were conducted in accordance with the Ordinance on the Prescription of Medicinal Products (Arzneimittelverschreibungsverordnung (§ 3a AMVV)) and the Ordinance on the Operation of Pharmacies (Apothekenbetriebsordnung, §17, Abs. 2b und 6b ApBetrO) as well as the Announcements of the Federal Institute for Drugs and medical Devices (BfArM).

### Results

In one patient the bleeding stopped after four days, in the patients with anticoagulants the bleeding stopped within the first two weeks. None of the patients reported side effects. Endoscopic controls after six months showed a regression of ectatic capillaries down to minor residual states (Figure 2). One year after the end of treatment, the success of the therapy persisted. None of the patients had a relapse. No damage to the rectal mucosa occurred. No side effects were reported.

## Claims

1. A compound selected from the group consisting of thalidomide, thalidomide analogues and pharmaceutically acceptable salts thereof, for use in the treatment of hemorrhagic chronic radiation proctitis, wherein said treatment comprises rectally administering said compound to a patient.

2. The compound for use according to claim 1, wherein said compound is thalidomide or a pharmaceutically acceptable salt thereof.

3. The compound for use according to claim 1 or 2, wherein the hemorrhagic chronic radiation proctitis is **characterized by** rectal bleeding.

4. The compound for use according to any one of the preceding claims, wherein the hemorrhagic chronic radiation proctitis is not an inflammatory condition.

5. The compound for use according to any one of the preceding claims, wherein the tissue affected by the hemorrhagic chronic radiation proctitis does not show signs of inflammation.

6. The compound for use according to any one of the preceding claims, wherein said patient had suffered and or is suffering from prostate cancer, uterine cancer or cervical cancer.

7. The compound for use according to any one of the preceding claims, wherein the patient had received radiotherapy at least 8 weeks prior to the onset of the hemorrhagic chronic radiation proctitis.

8. The compound for use according to any one of the preceding claims, wherein the compound is administered in the form of a solution, suspension, spray, foam, emulsion, ointment, cream, paste, suppository, or gel.

9. The compound for use according to claim 8, wherein the compound is administered in the form of a suppository.

10. The compound for use according to claim 8 or 9, wherein the compound is formulated in a single unit dosage form further comprising at least one excipient.

11. The compound for use according to any one of the preceding claims, wherein the compound is administered at a dose from 5 mg per day to 100 mg per day.

12. The compound for use according to claim 11, wherein the compound is administered at a dose from 15 mg per day to 55 mg per day.

13. The compound for use according to any one of the preceding claims, wherein the treatment period lasts from 3 to 70 days, or from 4 to 56 days, or from 5 to 42 days, or from 6 to 28 days, or from 7 to 21 days.

14. The compound for use according to any one of the preceding claims, wherein the patient receives one dose per day.

15. The compound for use according to any one of the preceding claims, wherein the patient is further treated with at least one anticoagulant.

## Amended claims

### Amended claims in accordance with Rule 137(2) EPC.

1. A compound for use in the treatment of hemorrhagic chronic radiation proctitis, wherein said compound is administered rectally to a patient, and wherein said compound is thalidomide or a pharmaceutically acceptable salt thereof.

2. The compound for use according to claim 1, wherein the hemorrhagic chronic radiation proctitis is **characterized by** rectal bleeding.

3. The compound for use according to any one of the preceding claims, wherein the hemorrhagic chronic radiation proctitis is not an inflammatory condition.

4. The compound for use according to any one of the preceding claims, wherein the tissue affected by the hemorrhagic chronic radiation proctitis does not show signs of inflammation.

5. The compound for use according to any one of the preceding claims, wherein said patient had suffered and or is suffering from prostate cancer, uterine cancer or cervical cancer.

6. The compound for use according to any one of the preceding claims, wherein the patient had received radiotherapy at least 8 weeks prior to the onset of the hemorrhagic chronic radiation proctitis.

7. The compound for use according to any one of the preceding claims, wherein the compound is administered in the form of a solution, suspension, spray, foam, emulsion, ointment, cream, paste, suppository, or gel.

8. The compound for use according to claim 7, wherein the compound is administered in the form of a suppository.

9. The compound for use according to claim 7 or 8, wherein the compound is formulated in a single unit dosage form further comprising at least one excipient.

10. The compound for use according to any one of the preceding claims, wherein the compound is administered at a dose from 5 mg per day to 100 mg per day.

11. The compound for use according to claim 10, wherein the compound is administered at a dose from 15 mg per day to 55 mg per day.

12. The compound for use according to any one of the preceding claims, wherein the treatment period lasts from 3 to 70 days, or from 4 to 56 days, or from 5 to 42 days, or from 6 to 28 days, or from 7 to 21 days.

13. The compound for use according to any one of the preceding claims, wherein the patient receives one dose per day.

14. The compound for use according to any one of the preceding claims, wherein the patient is further treated with at least one anticoagulant.
